# EUROPÄISCHE PATENTSCHRIFT

(11) **EP 2 005 994 B1**
(45) Veröffentlichungstag und Bekanntmachung des Hinweises auf die Patenterteilung: **17.08.2011**
(21) Anmeldenummer: 07110057.2
(22) Anmeldetag: 12.06.2007
(51) Int. Cl.: A61P 3/04, A23G 3/48, A23G 3/52, A23L 1/30

(54) **Funktionelles Nahrungsprodukt**
Functional foodstuff product
Produit alimentaire fonctionnel

(43) Veröffentlichungstag der Anmeldung: 24.12.2008
(73) Patentinhaber: SOFTSLIM GmbH, 1230 Wien (AT)
(72) Erfinder: Pöchhacker, Klaus, 1230 Wien (AT)
(74) Vertreter: Zech, Stefan Markus

(56) Entgegenhaltungen:
- US-A1- 2003 059 501
- US-A1- 2005 267 221
- US-A1- 2006 251 608
- DATABASE BIOSIS [Online] BIOSCIENCES INFORMATION SERVICE, PHILADELPHIA, PA, US; 10. Juli 2004 (2004-07-10), CHATTOPADHYAY ISHITA ET AL: "Turmeric and curcumin: Biological actions and medicinal applications" XP002463612 Database accession no. PREV200400406810

## Beschreibung

Die Erfindung betrifft ein funktionelles Nahrungsprodukt, insbesondere in Form eines Kaubonbons, umfassend eine erste Wirksubstanz in Form eines Curcuma-Extrakts. Curcuma oder Gelbwurz ist als krautartige Pflanze in der menschlichen Nahrung bekannt.

Es ist auch bekannt, Curcumin zur Steigerung der Fettoxidation und somit zur Gewichtsreduzierung zu verwenden (US 2005 026 7221).

Die Aufgabe der vorliegenden Erfindung besteht darin, ein funktionales Nahrungsprodukt unter Verwendung von Curcuma-Extrakt als Wirksubstanz zu schaffen.

Diese Aufgabe wird mit einen funktionellen Nahrungsprodukt nach den Merkmalen von Patentanspruch 1 gelöst. Es wird dabei ein funktionelles Nahrungsprodukt, insbesondere in Form eines Kaubonbons vorgeschlagen, wobei dieses funktionelle Nahrungsprodukt eine erste Wirksubstanz in Form eines Curcuma-Extrakts, eine zweite Wirksubstanz, die als funktionelle Form des L-Carnitins vorliegt, sowie ein mit Hilfe eines Expandiermittels aufschäumbares Trägermittel, insbesondere Geliermittel umfasst, wobei das aufschäumbare Trägermittel zusammen mit erster und zweiter Wirksubstanz unter Schutzgasatmosphäre zur Herstellung eines oral einnehmbaren Wirkstoffkonglomerates aufgeschäumt ist.

Dabei kann das funktionelle Nahrungsprodukt eine Diät bzw. Gewichtsabnahme unterstützen, wobei einerseits der beinhaltete Curcuma-Extrakt eine Appetitzügelung und das weiterhin enthaltene L-Carnitin eine erhöhte Fettverbrennung fördern und unterstützen.

In einer bevorzugten Ausgestaltung wird beim erfindungsgemäßen Nahrungsprodukt als Geliermittel Agar-Agar, Guar, Gummi Arabicum oder Inulin einzeln, in Kombination oder unter Kombination als Geliermittel eingesetzt.

Als Aufschäummittel finden bevorzugtermaßen CO₂ oder N₂ Verwendung.

In einer weiter bevorzugten Ausgestaltung umfasst das oral einnehmbare Nahrungsprodukt eine Vielzahl von unter Einwirkung des Aufschäummittels gebildeten Hohlräumen, so dass auch innerhalb des funktionellen Nahrungsproduktes große Flächen vorliegen, an denen die Wirksubstanz zur Aufnahme in den menschlichen Körper schnell verfügbar ist.

Weiters kann das Nahrungsprodukt noch Maltitsirup und/oder Isomalt und/oder weitere Zutaten umfassen.

Bevorzugtermaßen sind Bestandteile der ersten Wirksubstanz an Nanocarier (Größenordnung 1 bis 100 nm) gebunden. Dies ermöglicht insbesondere eine Aufnahme durch die Monozyten. Ein Teil der Monozyten wandert ins Gehirn in die Mikrogliazellen. In diesen wird die Bildung von NO-Synthetase induziert. Diese spaltet NO von Arginin ab. Ein weiterer Effekt ist die Bildung von Corticotropin releasing Factor (CRF) im Nucleus paraventricularis und die Ausschüttung von Dopamin im lateralen Hypothalamus. Dopamin reduziert das Hungerempfinden und den Appetit. Parallel hemmt CRF die Magensaftsekretion und dadurch ebenfalls den Appetit. CRF wird im Hypothalamus gebildet und unterstützt die Bildung der körpereigenen Opioide Beta-Endorphin und Dynorphin. Dadurch wird die Aufnahme fetthaltiger Nahrung reduziert. CRF fördert die Ausschüttung von ACTH (Adrenocorticotropes Hormon), dieses wiederum die Bildung von DHEAS (Dehydroepiandrosteron-Sulfat). Letztes hat einen deutlichen Einfluss auf die Bildung wirksamer Neurotransmitter und Modulatoren für die zentrale Steuerung von Hunger und Appetit.

Bevorzugtermaßen liegt die erste Wirksubstanz in Form des Curcuma-Extrakts in einer Dosierung von 3,6 x 10⁻² Gew. % bis 3,6 Gew. % vor, wobei dies eine sinnvolle Tagesdosierung sein kann und eine Dosierung insbesondere auf zweimal oder dreimal täglich aufgeteilt sein kann, so dass das einzelne Nahrungsprodukt dann eine um einen entsprechenden Faktor geringere Dosierung aufweisen würde. In Absolutwerten könnte Curama-Extrakt pro Einzeldosierung (z. B. Kaubonbon) in einer Dosierung von 1 mg bis 100 mg, bevorzugterweise von 5 mg bis 30 mg, besonders bevorzugt von etwa 15 mg vorliegen.

Die zweite Wirksubstanz als funktionelle Form des L-Carnitins liegt bevorzugtermaßen in einer Dosierung von 0,35 Gew. % bis 35 Gew. % vor. Auch hier handelt es sich bevorzugtermaßen um die empfohlene Tagesdosis, wobei bei einer Aufteilung dieser Dosis auf insbesondere zweimal oder dreimal täglich das einzelne Nahrungsprodukt eine um einen entsprechenden Faktor geringere Dosierung aufweisen würde. In Absolutwerten könnte das L-Carnitin pro Einzeldosierung (z. B. Kaubonbon) in einer Dosierung von 10 mg bis 1000 mg, bevorzugterweise von 50 mg bis 500 mg, besonders bevorzugt von etwa 200 mg vorliegen.

In einer konkret bevorzugten Ausgestaltung liegt das Nahrungsprodukt in Form eines Kaubonbons vor und ist einzeln oder zusammen mit weiteren Kaubonbons in einer Aluminiumverbundfolie, vorzugsweise unter Schutzgas, eingeschweißt.

Nach einem weiter bevorzugten Aspekt der vorliegenden Erfindung wird durch das Aufschäumen eine Volumenvergrößerung gegenüber der Ausgangsmasse um 20 % bis 300 % erzielt.

Das funktionelle Nahrungsprodukt kann synergistisch mehrere Wirkungen aufweisen, konkret nämlich die Appetitzügelung unterstützen und/oder die Fettverbrennung fördern.

In einem konkreten Ausführungsbeispiel liegt das Nahrungsprodukt als weiches Kaubonbon vor, das jeweils ein Gesamtgewicht von etwa 2,8 g aufweist und mit speziellen Aromen, wie beispielsweise Ananasaroma geschmacklich abgestimmt ist. Hierbei sind 0,54 Gew. % an Curcuma-Extrakt, also 15 mg Curcuma-Extrakt pro Kaubonbon vorgesehen. An zweiter Wirksubstanz in Form von L-Carnitin sind 7,14 Gew. %, also 200 mg pro Kaubonbon, vorgesehen. Gute Resultate haben sich eingestellt, wenn ein solches Kaubonbon mit der oben angegebenen bevorzugten Dosierung in einer Tagesdosis pro 30 kg Körpergewicht vorgesehen wird, das heißt eine 90 kg schwere Person täglich drei solcher Kaubonbons einnimmt.

Es ist im Ergebnis ein Nahrungsprodukt geschaffen, bei dem erste und zweite Wirksubstanz in aufeinander abgestimmter Weise zusammenwirken. Die erste Wirksubstanz in Form eines Curcuma-Extrakts wirkt generell als Appetitzügler, wobei diese Funktion durch das L-Carnitin als zweite Wirksubstanz unterstützt wird und gleichzeitig hierdurch die Fettverbrennung angeregt wird. Darüber hinaus fördert das L-Carnitin den Muskelaufbau und schafft dadurch wiederum "Brennöfen" für die Fettverbrennung.

Das erfindungsgemäße Nahrungsprodukt, insbesondere als Kaubonbon, hat weiterhin den Vorteil, dass die Wirkstoffkombination bereits über die Mundschleimhaut resorbiert wird und eine vollständige Resorption spätestens im Duodenum erfolgt.

Es kann jegliche geeignete funktionelle Form des L-Carnitins eingesetzt werden, beispielsweise das Salz des L-Carnitins, das L-Carnitin-Tartrat.

## Patentansprüche

1. Funktionelles Nahrungsprodukt, insbesondere in Form eines Kaubonbons, umfassend
- eine erste Wirksubstanz in Form eines Curcuma-Extrakts
- eine zweite Wirksubstanz, die als funktionelle Form des L-Carnitins vorliegt, sowie
- ein mit Hilfe eines Expandiermittels aufschäumbares Trägermittel, insbesondere Geliermittel,
wobei das aufschäumbare Trägermittel zusammen mit erster und zweiter Wirksubstanz unter Schutzgasatmosphäre zur Herstellung eines oral einnehmbaren Wirkstoffkonglomerates aufgeschäumt ist.

2. Nahrungsprodukt nach Anspruch 1,
**dadurch gekennzeichnet,**
**dass** das Geliermittel aus Agar-Agar und/oder Guar und/oder Gummi Arabicum und/oder Inulin gebildet ist.

3. Nahrungsprodukt nach Anspruch 1 oder 2,
**dadurch gekennzeichnet,**
**dass** als Aufschäummittel CO₂ oder N₂ Verwendung finden.

4. Nahrungsprodukt nach einem oder mehreren der Ansprüche 1 bis 3,
**dadurch gekennzeichnet,**
**dass** das oral einnehmbare Nahrungsprodukt eine Vielzahl von unter Einwirkung des Aufschäummittels gebildeten Hohlräumen umfasst.

5. Nahrungsprodukt nach einem oder mehreren der Ansprüche 1 bis 4,
**dadurch gekennzeichnet,**
**dass** das Nahrungsprodukt weiterhin Maltitsirup und/oder Isomalt umfasst.

6. Nahrungsprodukt nach einem oder mehreren der Ansprüche 1 bis 5,
**dadurch gekennzeichnet,**
**dass** die erste Wirksubstanz an Nanocarier (Größenordnung 1 bis 100 nm) gebunden ist.

7. Nahrungsprodukt nach einem oder mehreren der vorhergehenden Ansprüche,
**dadurch gekennzeichnet,**
**dass** die erste Wirksubstanz in Form des Curcuma-Extrakts in einer Dosierung von 3,6 x 10⁻² Gew. % bis 3,6 Gew. % vorliegt.

8. Nahrungsprodukt nach einem oder mehreren der vorhergehenden Ansprüche,
**dadurch gekennzeichnet,**
**dass** die zweite Wirksubstanz als funktionelle Form des L-Carnitins in einer Dosierung von 0,35 Gew. % bis 35 Gew. % vorliegt.

9. Nahrungsprodukt nach einem oder mehreren der vorhergehenden Ansprüche,
**dadurch gekennzeichnet,**
**dass** es in Form eines Kaubonbons vorliegt und einzeln oder zusammen mit weiteren Kaubonbons in einer Aluminiumverbundfolie, vorzugsweise unter Schutzgas, eingeschweißt ist.

10. Nahrungsprodukt nach einem oder mehreren der Ansprüche 1 bis 9,
**dadurch gekennzeichnet,**
**dass** durch das Aufschäumen eine Volumenvergrößerung gegenüber der Ausgangsmasse um 20 % bis 300 % erzielt wird.

11. Nahrungsprodukt nach einem oder mehreren der vorhergehenden Ansprüche zur Verwendung als Appetitzügler und/oder Fettverbrenner.

## Claims

1. A functional food product, in particular in the form of a chewing sweet, comprising
- a first active substance in the form of a curcuma extract,
- a second active substance which is present as a functional form of L-carnitine, as well as
- a carrier, in particular a gelling agent which is foamable by means of an expanding agent,
wherein the foamable carrier is foamed together with the first and second active substances in an inert gas atmosphere for the production of an orally ingestible active agent conglomerate.

2. The food product according to claim 1,
**characterized in that**
the gelling agent is formed of agar-agar and/or guar gum and/or gum Arabic and/or inulin.

3. The food product according to claim 1 or 2,
**characterized in that**
CO₂ or N₂ are used as expanding agents.

4. The food product according to one or more of claims 1 to 3,
**characterized in that**
the orally ingestible food product has a large number of cavities formed under the action of the expanding agent.

5. The food product according to one or more of claims 1 to 4,
**characterized in that**
the food product further comprises maltitol syrup and/or isomalt.

6. The food product according to one of more of claims 1 to 5,
**characterized in that**
the first active substance is bound to nanocarriers (size 1 to 100 nm).

7. The food product according to one or more of the preceding claims,
**characterized in that**
the first active substance in the form of the curcuma extract is present in a dosage of 3.6 x 10⁻² % by weight to 3.6 % by weight.

8. The food product according to one or more of the preceding claims,
**characterized in that**
the second active substance as a functional form of L-carnitine is present in a dosage of 0.35 % by weight to 35 % by weight.

9. The food product according to one or more of the preceding claims,
**characterized in that**
it is in the form of a chewing sweet and is sealed separately or together with further chewing sweets in an aluminum compound foil, preferably under inert gas.

10. The food product according to one or more of the claims 1 to 9,
**characterized in that**
the expanding achieves an increase in volume by 20 % to 300 % as compared to the initial mass.

11. The food product according to one or more of the preceding claims for use as an appetite suppressant and/or fat burner.

## Revendications

1. Produit alimentaire fonctionnel, en particulier sous forme d'un bonbon à mâcher, comportant
- une première substance active sous forme d'un extrait de curcuma,
- une deuxième substance active qui est présente comme forme fonctionnelle de la carnitine L, ainsi que
- un support pouvant mousser à l'aide d'un agent expanseur, en particulier un gélifiant,
où le support pouvant mousser est moussé conjointement avec la première et la deuxième substance active sous atmosphère de gaz protecteur pour la fabrication d'un conglomérat de principes actifs ingérable par voie orale.

2. Produit alimentaire selon la revendication 1,
**caractérisé en ce que**
le gélifiant est constitué d'agar-agar et/ou de guar et/ou de gomme arabique et/ou d'inuline.

3. Produit alimentaire selon la revendication 1 ou 2,
**caractérisé en ce que**
du CO₂ ou du N₂ sont employés comme agent moussant.

4. Produit alimentaire selon l'une ou plusieurs des revendications 1 à 3,
**caractérisé en ce que**
le produit alimentaire ingérable par voie orale comporte une multitude de cavités constituées sous l'effet de l'agent moussant.

5. Produit alimentaire selon l'une ou plusieurs des revendications 1 à 4,
**caractérisé en ce que**
le produit alimentaire comporte en outre du sirop de maltitol et/ou de l'isomalte.

6. Produit alimentaire selon l'une ou plusieurs des revendications 1 à 5,
**caractérisé en ce que**
la première substance active est liée à des nanoporteurs (de l'ordre de 1 à 100 nm de dimension).

7. Produit alimentaire selon l'une ou plusieurs des revendications précédentes,
**caractérisé en ce que**
la première substance active est présente sous forme de l'extrait de curcuma dans un dosage de 3,6 x 10⁻² % en poids à 3,6 % en poids.

8. Produit alimentaire selon l'une ou plusieurs des revendications précédentes,
**caractérisé en ce que**
la deuxième substance active est présente comme forme fonctionnelle de la carnitine L dans un dosage de 0,35 % en poids à 35 % en poids.

9. Produit alimentaire selon l'une ou plusieurs des revendications précédentes,
**caractérisé en ce que**
il est présent sous forme de bonbon à mâcher et est emballé seul ou avec d'autres bonbons à mâcher dans une feuille composite d'aluminium, de préférence sous gaz protecteur.

10. Produit alimentaire selon l'une ou plusieurs des revendications 1 à 9,
**caractérisé en ce que**
une augmentation de volume comprise entre 20 % et 300 % par rapport à la masse de départ est obtenue par le moussage.

11. Produit alimentaire selon l'une ou plusieurs des revendications précédentes pour utilisation comme coupe-faim et/ou brûleur de graisse.
